# EUROPEAN PATENT SPECIFICATION

(11) **EP 1 187 603 B1**
(45) Date of publication and mention of the grant of the patent: **08.08.2007**
(21) Application number: 00939884.3
(22) Date of filing: 14.06.2000
(51) Int. Cl.: A61K 31/137, A61K 31/352, A61P 3/04

(54) **COMBINATION THERAPY FOR EFFECTING WEIGHT LOSS AND TREATING OBESITY**
KOMBINATIONSTHERAPIE ZUR GEWICHTSREDUZIERUNG UND FETTLEIBIGKEITSBEHANDLUNG
THERAPIE COMBINEE PERMETTANT DE PERDRE DU POIDS ET DE TRAITER L'OBESITE

(30) Priority: 14.06.1999 US 139022 P; 26.01.2000 US 178563 P; 09.02.2000 US 181265 P
(43) Date of publication of application: 20.03.2002
(60) Divisional application: 07011472.3
(73) Proprietor: Vivus, Inc., Mountain View, CA 94040 (US)
(72) Inventor: Najarian, Thomas, Belmont, MA 02178 (US)
(74) Representative: Crump, Julian Richard John
(86) International application number: PCT/US2000/016434
(87) International publication number: WO 2000/076493

(56) References cited:
- PRIVITERA M D: "Topiramate: A new antiepileptic drug" ANNALS OF PHARMACOTHERAPY 1997 UNITED STATES, vol. 31, no. 10, 1997, pages 1164-1173, XP009022534 ISSN: 1060-0280
- SHAPIRA N A ET AL: "TREATMENT OF BINGE-EATING DISORDER WITH TOPIRAMATE: A CLINICAL CASE SERIES" JOURNAL OF CLINICAL PSYCHIATRY, XX, XX, vol. 61, no. 5, May 2000 (2000-05), pages 368-372, XP000951555 ISSN: 0160-6689
- DATABASE DRUGU ON STN ACCESSION NUMMER 2000-28434 ZARATE C.A.: 'antipsychotic drug side effect issues in bipolar manic patients', XP002909623 & J. CLIN. PSYCHIATRY vol. 61, 2000, pages 52 - 61, SUPPL. 8

## Description

About 97 million adults in the United States are overweight or obese. The medical problems caused by overweight and obesity are serious, and often life-threatening and include diabetes, shortness of breath, gallbladder disease, hypertension, elevated blood cholesterol levels, cancer, arthritis, other orthopedic problems, reflux esophagitis (heartburn), snoring, sleep apnea, menstrual irregularities, infertility and heart trouble. Moreover, obesity and overweight substantially increase the risk of morbidity from hypertension, dyslipidemia, type 2 diabetes, coronary heart disease, stroke, gallbladder disease, osteoarthritis and endometrial, breast, prostate, and colon cancers. Higher body weights are also associated with increases in all-cause mortality. Most or all of these problems are relieved or improved by permanent significant weight loss. Longevity is likewise significantly increased by permanent significant weight loss.

Weight loss treatments vary depending, at least in part, on the degree of weight loss one is attempting to achieve in a subject as well as on the severity of overweight or obesity exhibited by the subject. For example, treatments such as low-fat diet and/or regular exercise are often adequate in cases where a subject is only mildly overweight. Such treatments can be enhanced by controlled use of over-the-counter appetite suppressants including caffeine, ephedrine and phenylpropanolamine (Acutrim®, Dexatrim®). Moreover, prescription medications including amphetamine, diethylpropion (Tenuate®), mazindol (Mazanor®, Sanorex®), phentermine (Fastin®, Ionamin®), phenmetrazine (Preludin®), phendimetrazine (Bontrol®, Plegine®, Adipost®, Dital®, Dyrexan®, Melfiat®, Prelu-2®, Rexigen Forte®), benphetamine (Didrex®) and fluoxetine (Prozac®) are often used in the treatment of seriously overweight and/or obese subjects or patients. However, such treatments, at best, result in only ~5-10% weight loss (when accompanied with diet and exercise). Moreover, most of these treatments ultimately prove inadequate because they are either dangerous, ineffective or quickly lose their anorexient effect.

At least one class of these prescription medications, the phentermines (Fastin®, Ionamin®), have been used as monotherapy in the treatment of obesity for about 30 years. The phentermines are members of a class of drugs known as the sympathomimetics for their ability to mimic stimulation of the central nervous system. The phentermines act on the hypothalamus, an appetite control center of the brain. Phentermine monotherapy can increase weight loss when used in combination with diet and exercise, as compared to diet and exercise alone. However, the drug loses effectiveness after about two weeks and, in fact, is not approved by the FDA for use beyond six weeks. Moreover, weight loss may not be permanent, especially after the drug is discontinued. Phentermine treatment is also associated with side effects including nervousness, irritability, headache, sweating, dry-mouth, nausea and constipation.

In general, available weight loss drugs have limited efficacy and some clinically significant side effects. Studies of the weight loss medications dexfenfluramine (Guy-Grand, B. et al. (1989) Lancet 2:1142-5), orlistat (Davidson, M.H. et al. (1999) JAMA 281:235-42), sibutramine (Bray, G.A. et al. (1999) Obes. Res. 7:189-98), and phentermine (Douglas, A. et al. (1983) Int. J. Obes. 7:591-5) have shown similar effectiveness. Studies for each demonstrated a weight loss of about 5% of body weight for drug compared with placebo. Other serious considerations limit the clinical use of these drugs. Dexfenfluramine was withdrawn from the market because of suspected heart valvulopathy, orlistat is limited by GI side effects, sibutramine can cause hypertension, and phentermine has limited efficacy.

Various combination therapies which include phentermine as one of the agents have been investigated and have met with mixed success. The phentermines were, up until around 1997, often prescribed along with fenfluramine (Pondimin®) or dexfenfluramine (Redux®), nicknamed "fen", as a combination therapy known as fenphen. Fenfleuramine is a potent releaser of serotonin from serotonergic neurons which acts on a cerebral appetite center. When combined with phentermine, fenfluramine had the effect of enhancing and extending the anorexient action of phentermine, However in 1997, the Food and Drug Administration ("FDA") asked manufacturers to withdraw Pondimin® and Redux® due to studies which strongly suggested that the drugs cause damage to the mitral valve of the heart and pulmonary hypertension.

More recently, it has been suggested that phentermine in combination with anti _ depressants is a potentially effective therapy for effecting weight loss, U.S. Patent No. 5,795,895. In particular, the anti-depressants suggested for use in this new combination therapy are members of a class of compounds known as selective serotonin reuptake inhibitors (SSRIs) which include fluoxetine (Prozac®), sertraline (Zoloft®), fluvoxamine maleate (Luvox®) and trazodone hydrochloride (Desyrel®). The combination therapy is also suggested to treat coexisting depression and/or obsessive-compulsive disorder.

Phentermine has also recently been tested in combination with Bupropion (Wellbutrin®) for the treatment of obesity. Bupropion is an antidepressant that inhibits dopamine reuptake, as compared to serotonin uptake. It is also used to treat Attention Deficit Disorder (ADHD), bipolar depression, chronic fatigue syndrome, cocaine addiction, nicotine addiction, and lower back pain. While Bupropion alone had a modest effect as a weight loss agent (when prescribed to patients following a 1200 calorie per day diet), patients receiving phentermine in combination with Bupropion experienced no greater weight loss than those receiving Bupropion alone. Moreover, Bupropion use has been associated with drug-induced seizures causing it to be removed from the market by the FDA for at least five years before its re-introduction in 1989.

Accordingly, there exists a need for new, more effective weight loss treatments which are accompanied by fewer adverse or undesirable side effects or less serious side effects. In particular, there exists a need for developing medical weight loss treatments which can potentially lower major endpoints such as death and/or myocardial infarction rates by directly treating obesity rather than treating the consequences of obesity *(e.g.,* diabetes, hypertension, hyperlipidemia), as is currently the practice.
Privitera MD, the Annals of Pharmacotherapy Vol. 31, No. 10, 1164-1173 (1997) reviews proposed mechanisms of action, clinical pharmacology, efficacy, safety and tolerability of the antiepileptic drug topiramate.

Shapira et al., J. Clin. Psychiatry 61: 5, 368-372 (May 2000) is a clinical case series of treatment of binge-eating disorder with topiramate.

### Summary of the Invention

The present invention features a novel therapy for effecting weight loss which involves treating a subject with a sympathomimetic agent in combination with an anticonvulsant sulfamate derivative such that the subject experiences weight loss. In one aspect, the sympathomimetic agent is a compound having anorectic activity (*e.g.*, amphetamine, methamphetamine, benzphetamine, phentermine, chlorphentermine, diethylpropran, phenmetrazine and phendimetrazine). Preferably, the syinpathomimetic agent is the drug phentermine (nicknamed "phen"). In another aspect, the anticonvulsant sulfamate derivative is the drug topiramate.

The combinations used in the present invention also are effective against symptoms associated with Syndrome X. Accordingly, in another aspect methods are disclosed for treating Syndrome X with a combination of a sympathomimetic agent and an anticonvulsant sulfamate derivative (*e.g*., phentermine and topiramate) such that at least one symptom associated with Syndrome X is affected. Moreover, the combination methods have been shown to have beneficial side effects, such as ameliorating sleep apnea and lowering blood pressure, blood glucose, blood lipid and Hgb AlC levels. Accordingly, methods are described for treating at least one side effect associated with obesity. Preferably, at least one side effect of obesity is treated with a combination of a sympathomimetic agent in combination with an anticonvulsant sulfamate derivative.

The invention features pharmaceutical compositions including therapeutically effective amounts of a sympathomimetic agent in combination with an anticonvulsant sulfamate derivative. Kits including the pharmaceutical compositions of the present invention are also featured (*e.g.*, kits including the compositions packaged in a daily dosing regimen).

### Detailed Description of the Invention

The present invention is directed to a novel combination for use in preparation of a medicament for effecting weight loss in a subject. In particular, methods are described which involve treating the subject with a therapeutically effective amount of a combination of a sympathomimetic agent (*e.g.*, phentermine or a phentermine-like compound) and an anticonvulsant sulfamate derivative (*e.g.,* topiramate). The methods are particularly useful for the treatment of overweight and/or obesity, as well as in the treatment of Syndrome X.

The phrase "therapeutically-effective amount" as used herein refers to the amount of an agent, compound, drug, composition or combination of the invention which is effective for producing some desired therapeutic effect upon administration to a subject or patient (*e.g.*, a human subject or patient). The phrase "administering to a subject" or "administering to a patient" refers to the process of introducing an agent, compound, drug, composition or combination of the invention into the subjects or patient's body via an art-recognized means of introduction (*e.g.*, orally, transdermally, *via* injection, *etc.*).

The term "sympathomimetic agent" is a term of art and refers to agents or compounds which "mimic" or alter stimulation of the sympathetic nervous system (*e.g.,* stimulates the peripheral nervous system) of an organism (*e.g.*, mimic the stimulation naturally effected by physical activity, psychological stress, generalized allergic reaction and other situations in which the organism is provoked).

Sympathomimetic agents including those for use in the present invention as well as their general clinical uses or effects are set forth in Table I.

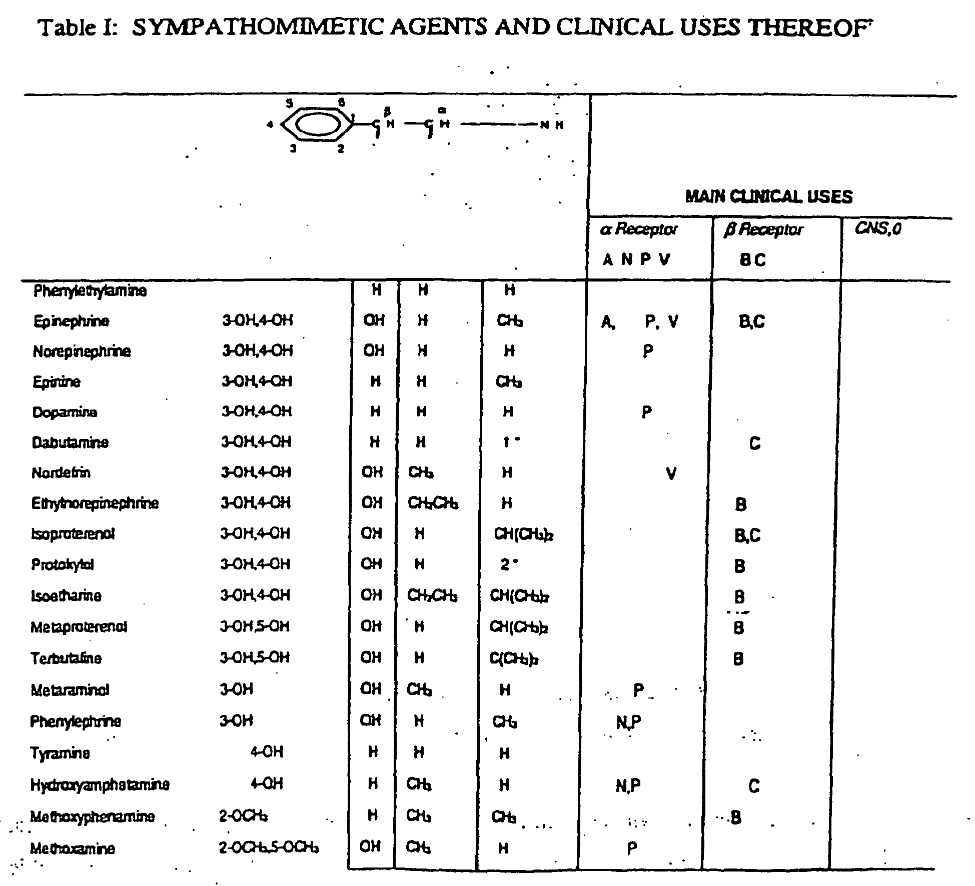

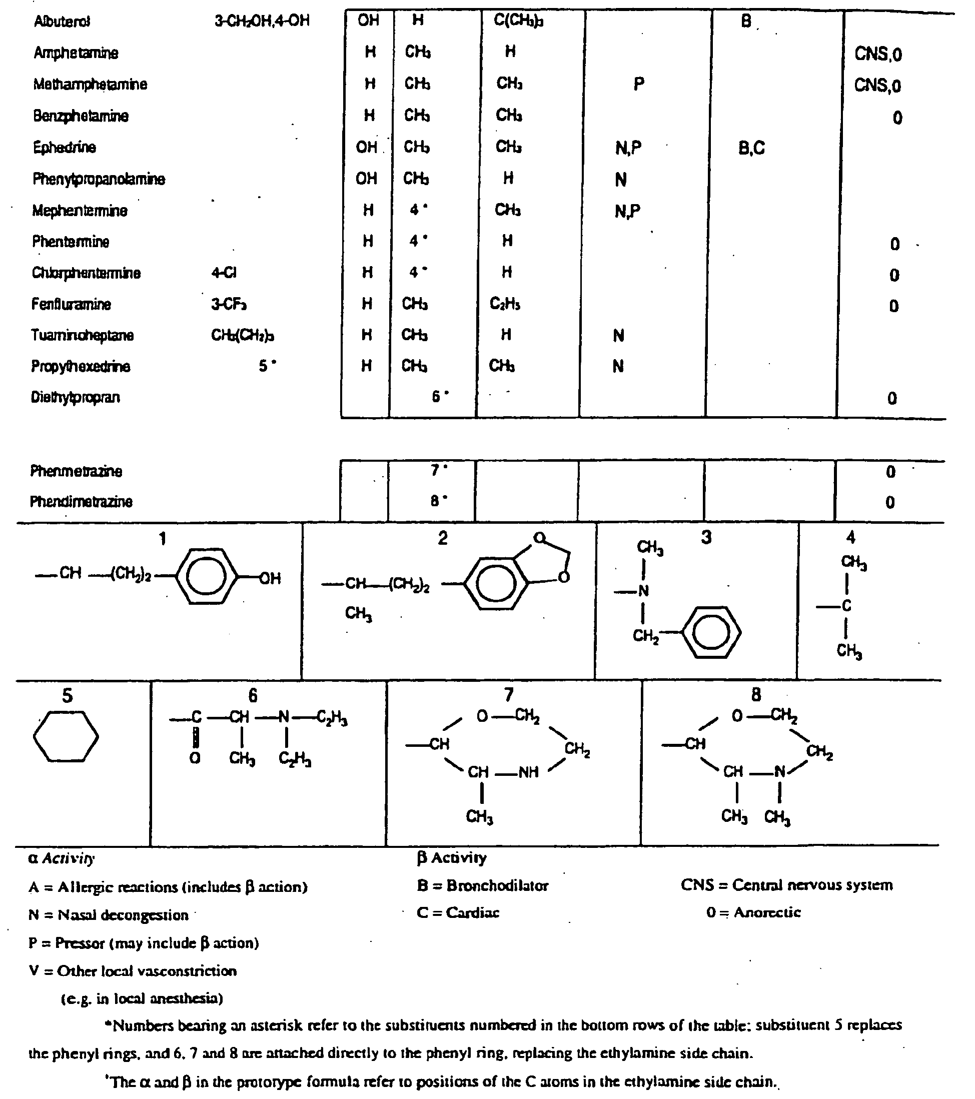

**In preferred embodiments, the sympathomimetic agent has anorexient properties** (*e.g.*, suppresses appetite) or is anorectic without significant toxicity to a subject or patient (*e.g.*, a human) at therapeutically effective doses. In a more preferred embodiment, the sympathomimetic agent has anorexient properties (*e.g.,* suppresses appetite) or is anorectic without loss of efficacy or without adverse or undesirable side effects to a subject or patient (*e.g.*, a human subject or patient) at therapeutically effective doses when prescribed in combination with topiramate. In yet another embodiment, the sympathomimetic agent is phentermine or a phentermine-like compound. As defined herein, a "phentermine-like compound" is a compound structurally related to phentermine (*e.g.*, an analog or derivative) which maintains an anorectic activity similar to phentermine. A preferred phentermine-like compound is chlorophentermine. In yet another embodiment, the sympathomimetic agent is amphetamine or an amphetamine-like compound. As used herein, an "amphetamine-like compound" is a compound structurally related to amphetamine (*e.g.*, an analog or derivative) which maintains an anorectic effect of amphetamine. In yet another embodiment, the sympathomimetic agent is phenmetrazine or a phenmetrazine-like compound. As defined herein, a "phenmetrazine-like compound" is a compound structurally-related to phenmetrazine (*e.g.*, an analog or derivative) which maintains the anorectic effect of phenmetrazine. A preferred phenmetrazine-like compound is phendimetrazine. Analogs and/or derivatives of the compounds of the present invention can be tested for their ability to suppress appetite (*e.g.,* suppress food intake) in a subject (eg., a mammalian subject).

In an exemplary preferred embodiment, the sympathomimetic agent is selected from the group consisting of amphetamine, methamphetamine, benzphetamine, phenylpropanolamine, phentermine, chlorphentermine, diethylpropran, phenmetrazine and phendimetrazine (as set forth in Table I. In a particularly preferred embodiment, the sympathomimetic agent is phentermine.

The terms "anticonvulsant sulfamate derivative" or "anticonvulsant sulfamate derivatives" are terms of art and refer to a class of sulfamate-derived compounds which possess anticonvulsant activity and have an art-recognized use in the treatment of epilepsy. In particular, the anticonvulsant sulfamate derivatives are monosaccharide derivatives with a sulfamate functionality. The anticonvulsant sulfamate derivatives for use in the present invention have one or more of the following modes of activity; modulation of voltage-dependent sodium conductance; potentiation of gamma-aminobutyric acid-evoked currents; inhibition of the kainate/alpha-amino-3-hydroxy-5-methylisoxazole-4-propionic acid (AMPA) subtype of the glutamate receptor; and/or inhibition of carbonic anhydrase *(e.g,* a mechanism by which the anticonvulsant derivative of the present invention may decrease the sensation of taste). Anticonvulsant sulfamate derivatives are described further in U.S. Patent Nos.: 4,513,006, 5,384,327, 5,498,629, 5,753,693 and 5,753,694, as are methods of synthesizing such anticonvulsant sulfamate derivatives.

The anticonvulsant sulfamate derivative is a compound having the following formula I: 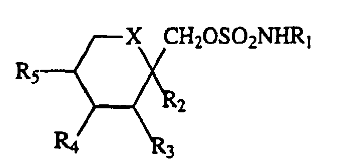 wherein
X is CH₂ or oxygen;
R₁ is hydrogen or alkyl; and
R₂, R₃, R₄ and R₅ are independently hydrogen or lower alkyl; when X is oxygen, R₂ and R₃ and/or R₄ and R₅ together may be a methylenedioxy group of the following formula II: 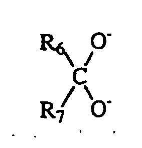 wherein
R₆ and R₇ are the same or different and are hydrogen, lower alkyl or are alkyl and are joined to form a cyclopentyl or cyclohexyl ring.
R₁ in particular is hydrogen or alkyl of 1 to 4 carbons, such as methyl, ethyl and iso-propyl. Alkyl includes both straight and branched chain alkyl. Alkyl groups R₂, R₃, R₄, R₅, R₆ and R₇ are 1 to 3 carbons and include methyl, ethyl, iso-propyl and n-propyl.

A particular group of compounds of formula I are those wherein X is oxygen and both R₂ and R₃, and R₄ and R₅ together are methylenedioxy groups of formula II, wherein R₆ and R₇ are both hydrogen, both alkyl, or combine to form a spiro cyclopentyl or cyclohexyl ring, in particular, where R₆ and R₇ are both alkyl such as methyl. A second group of compounds are those wherein X is CH₂ and R₄ and R₅ are joined to form a benzene ring. A third group of compounds of formula I are those wherein both R₂ and R₃ are hydrogen.

In preferred embodiments, the anticonvulsant sulfamate derivatives have anorexient properties (*e.g.,* suppress appetite) without significant toxicity to a subject or patient (*e.g.,* a human) at therapeutically effective doses. In a more preferred embodiment, the anticonvulsant sulfamate derivatives have anorexient properties *(e.g.,* suppress appetite) without significant adverse or undesirable side effects to a subject or patient *(e.g.,* a human) at therapeutically effective doses when prescribed in combination with phentermine. In a particularly preferred embodiment the anticonvulsant sulfamate derivative is topiramate (Topamax®). Topiramate, also referred to in the art as 2,3:4,5-bis-O-(1-methylethylidene)-β-D-fructopyranose sulfamate, has been demonstrated in clinical trials of human epilepsy to be effective as adjunctive therapy or as monotherapy in treating simple and complex partial seizures and secondarily generalized seizures (E. Faught et al. (1995) Epilepsia 36(suppl 4):33; S. Sachdeo et al. (1995) Epilepsia 36(suppl 4):33) and is currently marketed for the treatment of simple and complex partial seizure epilepsy with or without secondary generalized seizures.

### Dosages, Administration and Pharmaceutical Compositions

The choice of appropriate dosages for the drugs used in combination therapy according to the present invention can be determined and optimized by the skilled artisan, e.g., by observation of the patient, including the patient's overall health, the response to the combination therapy, and the like. Optimization, for example, may be necessary if it is determined that a patient is not exhibiting the desired therapeutic effect or conversely, if the patient is experiencing undesirable or adverse side effects that are too many in number or are of a troublesome severity.

Preferably, a sympathomimetic drug (*e.g.*, a drug set forth in Table I) is prescribed at a dosage routinely used by the skilled artisan *(e.g.,* physician) to promote the desired therapeutic effect of the drug when the drug is used as a monotherapy. Preferably, an anticonvulsant sulfamate derivative *(e.g.,* a compound having formula I) is prescribed at a lower dosage than routinely used by the skilled artisan (*e.g.*, physician) to promote the desired therapeutic effect of the drug, when the drug is used as a monotherapy (*e.g.,* in the treatment of epilepsy). In a preferred embodiment, a sympathomimetic drug or anticonvulsant sulfamate derivative is prescribed at a dose of between 5-1000, preferably between 10-1500, more preferably between 20-1000 and most preferably between 25-50 mg daily.

It is especially advantageous to formulate compositions of the invention in dosage unit form for ease of administration and uniformity of dosage. "Dosage unit form" as used herein refers to physically discrete units suited as unitary dosages for the individuals to be treated. Each dosage contains a predetermined quantity of an active compound calculated to produce the desired therapeutic effect in association with the required pharmaceutical carrier. The specification for the novel dosage unit forms of the invention is dependent on the unique characteristics of the composition containing the anticonvulsant or sympathomimetic agent and the particular therapeutic effect to be achieved. Dosages can further be determined by reference to the usual dose and manner of administration of the ingredients. It is also within the scope of the present invention to formulate a single physically discrete dosage form having each of the active ingredients of the combination treatment (*e.g.*, a single dosage form having anticonvulsant and sympathomimetic agent).

The method of administration of compositions or combinations of the invention will depend, in particular, on the type of sympathomimetic agent used and the chosen anticonvulsant sulfamate derivative. The sympathomimetic agent and the anticonvulsant sulfamate derivative may be administered together in the same composition or simultaneously or sequentially in two separate compositions. Also, one or more sympathomimetic agents or one or more anticonvulsant sulfamate derivatives may be administered to a subject or patient either in the form of a therapeutic composition or in combination, *e.g.,* in the form of one or more separate compositions administered simultaneously or sequentially. The schedule of administration will be dependent on the type of sympathomimetic agent(s) and anticonvulsant sulfamate derivative(s) chosen. For example, a sympathomimetic agent can have a stimulant effect and the degree of such stimulant effect may vary depending on the sympathomimetic agent chosen. Accordingly, a sympathomimetic agent having a significant stimulant effect might be administered earlier in the day than administration of a sympathomimetic agent having a lesser stimulant effect. Likewise, an anticonvulsant sulfamate derivative can have a sedative effect and the degree of such sedative effect may vary depending on the anticonvulsant sulfamate derivative chosen. Accordingly, an anticonvulsant sulfamate derivative having a significant sedative effect might be administered later in the day than administration of an anticonvulsant sulfamate derivative having a lesser sedative effect. Moreover, sympathomimetic agents and/or anticonvulsant agents having lesser stimulant or sedative effects, respectively, may be administered simultaneously.

Sympathomimetic agents and/or anticonvulsant sulfamate derivatives can also be administered along with a pharmaceutically-acceptable carrier. As used herein "pharmaceutically acceptable carrier" includes any solvents, dispersion media, coatings, antibacterial and antifungal agents, isotonic and absorption delaying agents, and the like. The use of such media and agents for pharmaceutically active substances is well known in the art. Except insofar as any conventional media or agent is incompatible with the active compound, use thereof in compositions of the invention is contemplated.

A sympathomimetic agent in combination with an anticonvulsant sulfamate derivative, is preferably administered orally. When the composition(s) are orally administered, an inert diluent or an assimilable edible carrier may be included. The composition and other ingredients may also be enclosed in a hard or soft shell gelatin capsule, compressed into tablets, or incorporated directly into the individual's diet. For oral therapeutic administration, the composition may be incorporated with excipients and used in the form of ingestible tablets, buccal tablets, troches, capsules, elixirs, suspensions, syrups, wafers, and the like. The percentage of the compositions and preparations may, of course, be varied. The amount of active compound in such therapeutically useful compositions is such that a suitable dosage will be obtained. In a particularly preferred embodiment, the present invention includes a pharmaceutical composition comprising a therapeutically-effective amount of a sympathomimetic agent and an anticonvulsant sulfamate derivative. In one embodiment, the present invention includes a therapeutically-effective amount of a sympathomimetic agent and an anticonvulsant sulfamate derivative packaged in a daily dosing regimen (*e.g.*, packaged on cards, packaged with dosing cards, packaged on blisters or blow-molded plastics, etc.). Such packaging promotes products and increases patient compliance with drug regimens. Such packaging can also reduce patient confusion. The present invention also features such kits further containing instructions for use.

The tablets, troches, pills, capsules and the like may also contain a binder, an excipient, a lubricant, or a sweetening agent. Various other materials may be present as coatings or to otherwise modify the physical form of the dosage unit. For instance, tablets, pills, or capsules may be coated with shellac, sugar or both. Of course, any material used in preparing any dosage unit form should be pharmaceutically pure and substantially non-toxic in the amounts employed.

A sympathomimetic agent in combination with an anticonvulsant sulfamate derivative, can also be administered in a convenient manner such as by injection (subcutaneous, intravenous, etc.), inhalation, transdermal application, or rectal administration. Depending on the route of administration, the composition containing the sympathomimetic agent and/or anticonvulsant sulfamate derivative may be coated with a material to protect the compound from the action of acids and other natural conditions which may inactivate the compounds or compositions.

To administer the compositions, for example, transdermally or by injection, it may be necessary to coat the composition with, or co-administer the composition with, a material to prevent its inactivation. For example, the composition may be administered to an individual in an appropriate diluent or in an appropriate carrier such as liposomes. Pharmaceutically acceptable diluents include saline and aqueous buffer solutions. Liposomes include water-in-oil-in-water CGF emulsions as well as conventional liposomes (Strejan *et al.* (1984) *J. Neuroimmunol.* 7:27). To administer the compositions containing the sympathomimetic agents and/or anticonvulsant sulfamate derivatives parenterally or intraperitoneally, dispersions can be prepared in glycerol, liquid polyethylene glycols, and mixtures thereof and in oils. Under ordinary conditions of storage and use, these preparations may contain a preservative to prevent the growth of microorganisms.

Compositions suitable for injectable use include sterile aqueous solutions (where water soluble) or dispersions and sterile powders for the extemporaneous preparation of sterile injectable solutions or dispersion. In all cases, the composition must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), suitable mixtures thereof, and vegetable oils. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents. In many cases, it will be preferable to include isotonic agents, for example, sugars, polyalcohols such as manitol, sorbitol, and/or sodium chloride in the composition. Prolonged absorption of the injectable compositions can be brought about by including in the composition an agent which delays absorption, for example, aluminum monostearate and gelatin.

Preferably phentermine is prescribed in combination with topiramate to effect weight loss and/or to treat Syndrome X and/or a subset of symptoms thereof. A preferred dose for phentermine is between 5-60 mg daily, including but not limited to doses of 8, 10, 15, 20, 25, 30, 35, 40, 45, 50 and 55 mg daily. A particularly preferred dose for phentermine is 15 mg daily. In an exemplary embodiment, the phentermine is of an immediate release form. Preferably, the phentermine is taken by the patient in the morning and more preferably, is taken before breakfast. The phentermine is best taken in the morning because the drug is a stimulant as well as an appetite suppressant: When phentermine is prescribed (*e.g.*, as part of the combination therapy described herein), physicians should be aware and may want to advise patients that the drug can be mildly habit forming. Phentermine can also cause increased.nervousness, increased energy, irritability and, rarely, insomnia. Stopping phentermine may also cause tiredness lasting for up to 1-2 weeks. Phentermine can also raise blood pressure (*e.g.*, during the early phases of treatment).

A preferred dose for topiramate is between 50-1500 mg daily. As discussed previously, prescription of topiramate at dosages of ≥ 400 mg daily results in promotion of undesirable side effects (*e.g.*, sedation, mental clouding). Accordingly, in a preferred embodiment, topiramate is prescribed at a dose of 50-400 mg daily. In another preferred embodiment, the dosage of topiramate is increased gradually at the outset of the therapy in order to reduce the chance of undesirable side effects associated with higher doses of the drug. In an exemplary embodiment, the topiramate is administered at a dose of 25 mg daily for the first 5-7 days *(e.g.,* 6 days) of treatment, at a dose of 50 mg daily for the next 5-7 days *(e.g.,* 6 days), at a dose of 100 mg daily for the next 6-8 days (*e.g.*, 7 days) and 150 mg daily for the next 20-26 days. From this point forward, the topiramate can be administered at a dose of 150-250 mg daily, including but not limited to doses of 175, 200 and 225 mg daily. A particularly preferred dose for continued therapy is 200 mg of topiramate daily. In another exemplary embodiment, the topiramate is of an immediate release form. In yet another exemplary embodiment, the topiramate is of a sustained release form.

In a preferred embodiment, topiramate is taken later in the day than the phentermine. Preferably, the patient takes the topiramate just before supper or later in the evening. Topiramate is best given later in the day because the drug can be sedating. In other embodiments, the topiramate is given BID (*e.g.*, twice daily), TID (three times daily) or QID (four time daily). When prescribing topiramate, physicians should be aware and may want to advise patients that the drug can cause tiredness, fatigue, dizziness, difficulty with speech or finding words, difficulty concentrating, difficulty with balance, and/or numbness or tingling in the hands or feet. Less common side effects are nausea, coordination problems, abdominal pain, slowed thinking, nervousness, depression, breast pain, painful periods, double or blurred vision, palpitations, low white blood count and kidney stones. A physician should also advise patients that the drug may not be taken if the patient is also taking Diamox (acetazolamide). No female patient should become pregnant while taking this drug as it may cause birth defects. If a female patient misses a period she should immediately discontinue taking the medication and inform the physician. Female patients should not be treated according to the methods of the present invention if breast feeding a child. Patients should not drink alcohol or take sedating medications while taking topiramate since excess sedation can occur. Patients should also refrain from performing dangerous tasks (e.g. operating heavy machinery or driving) until they are comfortable with the side effects of the full dose (*e.g.*, 200-400 mg daily). Patients should be advised not to increase the dosage beyond what is prescribed. Topiramate is not habit forming.

Yet another embodiment of the present invention features pharmaceutical compositions (*e.g.*, for oral administration) comprising phentermine and topiramate in a single pharmaceutical formulation. Such compositions may be preferred, for example, to increase patient compliance (*e.g.,* by reducing the number of administrations necessary to achieve the desired phamacologic effect.)

In a preferred embodiment, the pharmaceutical composition includes phentermine in an immediate release form and further includes topiramate in a controlled release formulation. As defined herein, an "immediate release formulation" is one which has been formulated to allow, for example, the phentermine, to act as quickly as possible. Preferred immediate release formulations include, but are not limited to, readily dissolvable formulations. As defined herein, a "controlled release formulation" includes a pharmaceutical formulation which has been adapted such that release rates and profiles can be matched to physiological and chronotherapeutic requirements or alternatively, has been formulated to effect release of a drug at a programmed rate. Preferred controlled release formulations include, but are not limited to, granules, delayed release granules, hydrogels (*e.g.*, synthetic or of natural origin), other gelling agents (*e.g.*, gel-forming dietary fibers), matrix-based formulations (*e.g.*, formulations comprising a polymeric marterial having at least one active ingredient dispersed therethrough), granules within a matrix, polymeric mixtures, granular masses, and the like.

In one embodiment, a controlled release formulation is a delayed release form. As defined herein, a "delayed release form" is formulated in such a way as to delay, for example, topiramate's action for an extended period of time. A delayed release form can be formulated in such a way as to delay the release of an effective dose of topiramate for 4, 8, 12, 16 or 24 hours following the release of phentermine. In yet another preferred embodiment, a controlled release formulation is a sustained release form. As defined herein, a "sustained release form" is formulated in such a way as to sustain, for example, the topiramate's action over an extended period of time. A sustained release form can be formulated in such a way as to provide an effective dose of topiramate (*e.g.*, provide a physiologically effective blood level) over a 4, 8, 12, 16 or 24 hour period.

Preferred compositions include a tablet core consisting essentially of topiramate, said core being in association with a layer of phentermine. Preferably, the core has a delayed or sustained dissolution rate. In an exemplary embodiment, a tablet can comprise a first layer containing, for example, phentermine (*e.g*., in an immediate release formulation) and a core containing, for example, topiramate in a delayed release or sustained release formulation. Other exemplary embodiments can include, for example, a barrier between the first layer and core, said layer serving the purpose of limiting the drug release surface of the core. Preferred barriers prevent dissolution of the core when the pharmaceutical formulation is first exposed to gastric fluid. For example, a barrier can comprise a disintegrant, a dissolution-retarding coating (*e.g.*, a polymeric material, for example, an enteric polymer), a hydrophobic coating or film, and can further be selectively soluble in either the stomach or intestinal fluids. Such barriers permit the topiramate to leach out slowly and can cover substantially the whole surface of the core.

The above-described pharmaceutical compositions are designed to release the two effective agents of the combination therapy of the present invention sequentially, *i.e.,* releasing topiramate after releasing phentermine, both agents being contained in the same pharmaceutical composition. Preferred amounts of phentermine and topiramate are as described above with particularly preferred compositions comprising from 5 mg to 60 mg phentermine and from 50 mg to 1500 mg topiramate. Particularly preferred compositions include at least 15 mg phentermine and at least 50 mg, 100 mg or 200 mg topiramate.

Pharmaceutical compositions so formulated may contain additional additives, suspending agents, diluents, binders or adjuvants, disintegrants, lubricants, glidants, stabilisers, coluring agents, flavours, etc. These are conventional materials which may be incorporated in conventional amounts.

A method may be carried out, practiced or performed such that weight loss in the subject or patient occurs. Accordingly, the methods are particularly useful for the treatment of overweight or obese patients. As defined herein, an overweight subject or patient is between 1 and 20 percent over weight *(e.g.,* weighs 1-20% in excess of their ideal body weight). Also as defined herein, an obese subject or patient is greater than 20 percent overweight (*e.g*., weighs >20% in excess of their ideal body weight). Alternatively, the methods are useful in the treatment of subjects or patients in need of losing weight, but which are not necessarily overweight or obese. For example, it may be desirable to achieve weight loss in subjects or patients having arthritis or prostheses such that the subject experiences less adverse effects resulting from bearing weight.

The combination therapies will generally be administered until the patient has experienced the desired weight loss, and preferably has achieved an ideal body weight. Alternatively, the combination therapies can be administered until the patient has achieved a weight loss of 5-10%, 10-15%, 15-20% or 20-25% of their initial body mass (*e.g*.**,** the patient's starting weight).

The present invention has also recognized that the combination therapy ameliorates symptoms associated with Syndrome X. Syndrome X consists of a complex of medical problems that are largely associated with obesity, including hypertension, diabetes or glucose intolerance and insulin resistance, hyperlipidemia, and often tiredness and sleepiness associated with sleep apnea. Patients are often treated with combinations of antihypertensives, lipid lowering agents, insulin or oral diabetic drugs, and various mechanical and surgical treatments of sleep apnea. However, such treatments are often costly and do not treat the underlying problem of obesity. Moreover, some of the treatments for diabetes including insulin and oral diabetic agents actually aggravate Syndrome X by increasing insulin levels, increasing appetite and increasing weight. This can lead to higher blood pressure and even higher cholesterol. Accordingly, a method is described of treating Syndrome X using the combination therapies described herein. A method of treating Syndrome X in a subject or patient is described which includes treating the subject with a therapeutically effective amount of a combination of an anticonvulsant sulfamate derivative *(e.g.,* topiramate) and a sympathomimetic agent (*e.g.,* phentermine or a phentermine-like compound), such that at least one symptom associated with Syndrome X is affected. As defined herein, "affecting a symptom" (*e.g.,* affecting a symptom associated with Syndrome X) refers to lessening, decreasing the severity of the symptom or reversing, ameliorating, or improving the symptom (*e.g.*, decreasing hypertension, ameliorating diabetes, reversing glucose intolerance or insulin resistance, lessening hyperlipidemia, or decreasing tiredness and sleepiness associated with sleep apnea).

Treatment of Syndrome X according to the methods described herein includes affecting at least one, preferably two, more preferably three, more preferably four, five or six symptoms associated with Syndrome X. Particularly preferably all symptoms associated with Syndrome X are affected *(e.g.,* lessened, reversed, ameliorated, etc.)

The present invention has also recognized that the combination therapy ameliorates some side effects associated with obesity, as described herein. Accordingly, a method is described of treating at least one side effect associated with obesity using the combination therapies described herein. A method is described of treating at least one obesity-related side effect in a subject or patient which includes treating the subject with a therapeutically effective amount of a combination of an anticonvulsant sulfamate derivative (*e.g.,* topiramate) and a sympathomimetic agent (*e.g*., phentermine or a phentermine-like compound), such that at least one obesity-related side effect is effected. As defined herein, a "side effect associated with obesity" includes a symptom or disorder in a subject *(e.g.,* a patient) which is secondary and/or results from *(e.g.,* directly and/or indirectly results from) a medical condition of obesity Preferably, the subject is obese and/or is being treated for obesity. The subject may have at least one or more (*e.g.*, two, three, four, five or more) side effect(s) selected from the group consisting of sleep apnea, high blood pressure and high blood sugar, high blood lipid, high Hgb A1C or other art-recognized side effects associated with obesity.

Whether in the treatment of Syndrome X or in the practicing of the methods to effect weight loss (*e.g.*, in the treatment of overweight and/or obesity) or in treatment of side effects associated with obesity, it will be apparent to the skilled artisan (*e.g.,* physician) that monitoring of the patient is needed to determine the effectiveness of the treatments and to potentially modify the treatments (*e.g.,* modify the dosing, time of drug administration, sequence of drug administration) as defined herein. Accordingly, in certain embodiments, the patient is monitored about every 2-6 weeks, preferably every 3-5 weeks and more preferably every 4 weeks. Monitoring the effectiveness of treatment to achieve weight loss includes, but is not limited to monitoring the subject or patient's body weight (*e.g.,* comparing the patient's initial body weight to that at a follow-up visit, for example, four weeks after the initiation of treatment). Additional features of the subject's or patient's health can also be monitored *(i.e.,* monitoring the patient's overall health and/or monitoring the effectiveness of treatment of an undesired side effect of obesity) including, but not limited to the patient's blood pressure, blood sugar, serum lipid levels, *etc*. Likewise, monitoring a subject or patient for treatment of Syndrome X can include monitoring of at least one, preferably more than one symptom associated with Syndrome X.

This invention is further illustrated by the following examples which should not be construed as limiting.

### Example 1:

Patients as part of the following trial are treated according to the following dosage regime. Patients take phentermine at a dose of 15 mg daily throughout the weight loss program. Patients take the phentermine before breakfast. For the first 6 days, patients take one 25 mg tablet of topiramate before supper. For the next 6 days, patients take two 25 mg tablets of topiramate before supper. For the next 7 days (days 13-19), patients take 100 mg before supper daily using 4-25 mg tablets of topiramate daily. For days 20-26, patients take 150 mg of topiramate daily consisting of one half of a 200 mg tablet and two 25 mg tablets of topiramate. From that point on, unless instructed otherwise by the physician, patients continue to take one 200 mg topiramate tablet daily before supper and continue the 15 mg phentermine daily in the morning. Patients were advised to drink at least eight (8) full glasses of water daily to reduce the risk of kidney stones which may result from taking topiramate.

Patients are advised that while the effect of phentermine is fairly rapid, the effect of topiramate is slower in onset. The weight reduction of topiramate will continue for as long as 18 months on the medication. That is, the patient can expect to continue gradual weight loss for up to 18 months on the medication. Of course, weight loss is maximal if the patient follows diet and/or exercise programs. The weight loss should exceed 15% of the patients starting weight. Thus, if the patient weighs about 90.7 kg (200 pounds) as of the start date, he/she might expect to lose at least 13.6 kg (30 pounds) in a period of 12-18 months. The following patient data has been collected.

| Patient's Initials | Age | Sex | Start Weight (lbs) [kgs] | Start Blood Pressure | Follow-Up Date | Follow-Up Weight (lbs) [kgs] | % Weight Loss | Follow-Up Blood Pressure |
|---|---|---|---|---|---|---|---|---|
| M.O. | 48 | F | 182 [82.6] | 115/70 | 5 weeks | 177 80.3 | 2.7% | 120/80 |
| | | | | | 9 weeks | 176 79.3 | 3.3% | 110/70 |
| T.M. | 37 | F | 190 [86.2] | 122/84 | 2 weeks | 178 80.7 | 6.3% | 110/80 |
| | | | | | 5 days | | | |
| | | | | | 6 weeks, | 168 76.2 | 11.6% | 125/80 |
| | | | | | 2 days | | | |
| D.M.(A) | 28 | M | 286 [129.7] | 138/90 | 4 weeks | 279 126.6 | 2.4% | 128/86 |
| P.L. | 55 | F | 144 [65.3] | 132/84 | 4 weeks | 141 64.0 | 2.1% | 138/85 |
| | | | | | 9 weeks | 137 62.1 | 4.9% | 122/82 |
| E.K. | 52 | F | 181 [82.1] | 130/100 | 5 weeks | 175 79.4 | 3.3% | 140/88 |
| I.F. | 41 | F | 196 [88.9] | 95/60 | 6 weeks, | | | |
| | | | | | 5 days | | | |
| D.M.(B)² | 56 | M | 295 [133.8] | 150/80 | 4 weeks. | 297 134.7 | (+0.7%) | 148/82 |
| | | | | | 2 days | | | |
| | | | | | 8 weeks, | 287 [130.2] | 2.7% | 140/70 |
| | | | | | 2 days | | | |

| | | | | | | | | |
|---|---|---|---|---|---|---|---|---|
| ¹ Patient M.O. was being treated with Meridia® at the onset of the study, which continued through the first 5 weeks of the study. At the 5-week follow up, M.O. was switched to the phentermine/topiramate regime described above. ² Patient D.M.(B) was being treated with phentermine alone at the onset of the study and was taking the full dose of topiramate by the fourth week of the study. | | | | | | | | |

As is apparent from the above-described data, patients not previously being treated with an anorexient at the outset of the study experienced an average of about 3.5% weight loss after only 2-6 weeks ***(e.g.,*** patient T.M. lost 6.3% body weight, patient D.M.(A) lost 2.4% body weight, patient P.L. lost 2.1% body weight and patient E.K lost 3.3% body weight). After only 6-9 weeks of treatment, patients *(e.g.,* those not previously being treated with an anorexient at the outset of the study) experienced an average of about 8.3% weight loss (*e.g.,* patient T.M. lost 11.6% body weight and patient P.L lost 4.9% body weight). The patient previously on Meridia® (patient M.O. lost 3.3% body weight after being enrolled in the program for 9 weeks). Moreover, the patient previously on phentermine (patient D.M.(B)) lost a total of 2.7% body weight after being enrolled in the program for about 8 weeks. This particular patient reported that this is the most significant weight loss he has achieved to date, the patient having previously tried other conventional therapies.

In addition to the weight loss reported above, almost all patients enrolled in the study experienced decreased blood pressure. Moreover, patients involved in the study who had previously taken Redux, phen-fen, Meridia and/or other weight loss treatments reported that they had not previously experienced the benefits of the combined phentermine/topiramate therapy. Patients reported that they had no appetite, could resist food easily, could concentrate and function at work (even in attention-intensive jobs such as computer programming), had more energy and felt better. Patients also reported experiencing less side effects than any previous weight loss treatments tried.

### Example 2:

Extended results of the trial described in Example 1.

A total of thirteen patients were treated for 1-9 months with phentermine (15 mg daily) in the morning and up to 400 mg of topiramate (median dose 200 mg), in the evening. [Note: Patient D.M.(B) discussed above is not included in this data as he was on phentermine treatment prior to treatment with the combination therapy of the present invention.] Topiramate dose was gradually increased from 25 mg per day in increments of 25-50 mg weekly until either desirable weight loss took place or until side effects limited dose increases. [Note: A fourteenth patient discontinued treatment after 3 days due to nausea.] All thirteen patients tolerated treatment well with minimal side effects. Along with taking medication, patients were instructed to walk at least 30 minutes three times per week and to follow a low fat diet. No patients had taken diet medication for at least 3 months prior to treatment. Average baseline BMI was 32.5 (range 26-48).

Average weight loss for the thirteen patients was 11.8%. For seven patients who were on treatment the longest (range 5-9 months), the average weight loss was 14.4%. Patients reported that they had little or no appetite and that they actually felt better (Topiramate's usefulness is also being investigated as a mood stabilizer) than before therapy. Blood pressure, lipid, glucose, and Hgb A1C values were also favorably affected by this treatment.

Table I sets forth patient data for the thirteen above-described patients treated with the combination therapy of the present invention.

**TABLE I**

| **PATIENT DATA: COMBINATION THERAPY*** | | | | | |
|---|---|---|---|---|---|
| **Patient No.** | **% of Weight Loss** | **Baseline BMI** | **Current BMI** | **Weeks on Rx** | **Current Status** |
| 1 | 7.7 | 38 | 35 | 10 | on Rx |
| 2 | 10.3 | 25 | 23 | 4 | Finished |
| 3 | 6.8 | 48 | 44 | 5 | d/c early - dropped out |
| 4 | 16.8 | 30 | 24 | 35 | on taper |
| 5 | 23.2 | 30 | 23 | 41 | on taper |
| 6 | 8 | 41 | 38 | 40 | on Rx |
| 7 | 9.7 | 28 | 25 | 33 | on taper |
| 8 | 14.4 | 30 | 26 | 44 | on Rx |
| 9 | 15.9 | 27 | 21 | 32 | on taper |
| 10 | 9 | 33 | 31 | 7 | d/c early - will restart |
| 11 | 12.9 | 28 | 24 | 22 | Finished |
| 12 | 7 | 29 | 27 | 5 | on Rx |
| 13 | 12.1 | 34 | 31 | 6 | on Rx |

| | | | | | |
|---|---|---|---|---|---|
| * data for thirteen patients | | | | | |

Average weight loss = 11.8% (13 patients)
Average weight loss ≥ 22 weeks on Rx = 14.4% (7 patients)
Average baseline BMI = 32.4

Table II sets forth the average blood pressure, blood glucose, Hgb A I C and blood lipid value for the thirteen patients.

**TABLE II**

| | **BP** | **GLUCOSE** | **HGBA1C** | **CHOL** | **TRIG** |
|---|---|---|---|---|---|
| | **mmHg** | **mg/dL** | **%*** | **mg/dL** | **mg/dL** |
| Average Pre-Treatment | 131.3/ | 107 | 6.48 | 212 | 189 |
| Value | 85.9 | | | | |
| Average On Treatment | 122.6/ | 102 | 5.05 | 210 | 172 |
| Value | 78.4 | | | | |

| | | | | | |
|---|---|---|---|---|---|
| * Numbers include 1 diabetic patient whose oral hypoglycemic was reduced by 50% while on the weight loss treatment. | | | | | |

One of the thirteen patients in the study also had severe sleep apnea with the usual complications of daytime sleepiness and fatigue. His symptoms have disappeared with the weight loss treatment.

Of the six patients who have completed (*i.e.*, finished or on taper) the combination therapy of the present invention, five of the six achieved a body mass index (BMI) of 24 or better. The average pre-treatment or baseline BMI for these six patients was 28. The final average BMI was 23.3. The average weight loss was 1'7%.

### Example 3:

The 56-year old male patient described previously (D.M.(B)) who was initially taking phentermine alone and had topiramate added to his regimen had a good effect from the combination. He once weighed as much as 179.2 kg (395 pounds). When Redux was still on the mark in the United States, he was treated with a combination of diet, exercise, Redux and phentermine. His lowest weight attained was 129.3 kg (285 pounds). When Redux was withdrawn from the market, he remained on phentermine but gained weight back to 133.8-136.1 kg (295-300 pounds) When topiramate was added to his regimen, he managed to lose 11.3 kg (25 pounds) and is currently at 127,9 kg (271 pounds) his lowest weight since he was in his 20s. He, along with most of the patients treated so far, reported that the treatment with topiramate and phentermine had less side effects and was more effective than any previous weight loss treatment using medications that he and others had tried. This 56-year old man exhibited lowered blood pressure (approx.15 mm Hg systolic and 10 mm Hg diastolic).

### Example 4:

Extended results of the trial described in Examples 1 and 2.

The cumulative data from a total of seventeen patients treated with the combination weight loss treatment of the present invention are set forth in Table III.

**TABLE III**

| **PATIENT DATA: COMBINATION THERAPY*** | | | | | |
|---|---|---|---|---|---|
| **PATIENT** | **% WEIGHT LOSS** | **BASELINE BMI** | **CURRENT BMI** | **WEEKS ON Rx** | **CURRENT STATUS** |
| 1 | 7.7 | 38 | 35 | 33 | on Rx |
| 2 | 10.3 | 25 | 23 | 4 | Finished |
| 3 | 6.8 | 48 | 44 | 5 | d/c early-dropped out |
| 4 | 16.8 | 30 | 24 | 58 | on taper |
| 5 | 23.2 | 30 | 23 | 64 | on taper |
| 6 | 17.5 | 41 | 33 | 63 | on Rx |
| 7 | 9.7 | 28 | 25 | 56 | on taper |
| 8 | 18.6 | 30 | 24 | 67 | on Rx |
| 9 | 15.9 | 27 | 21 | 55 | on taper |
| 10 | 9 | 33 | 31 | 7 | d/c early-will restart |
| 11 | 12.9 | 28 | 24 | 22 | Finished |
| 12 | 7 | 29 | 27 | 5 | d/c early-will restart |
| 13 | 12.1 | 34 | 31 | 6 | d/c early-will restart |
| 14 | 22.5 | 46 | 32 | 16 | on Rx |
| 15 | 10.1 | 50 | 45 | 12 | on Rx |
| 16 | 6.4 | 27 | 24 | 4 | Finished |
| 17 | 6.3 | 27 | 25 | 6 | on Rx |

| | | | | | |
|---|---|---|---|---|---|
| *data for seventeen patients | | | | | |

AVERAGE WEIGHT LOSSS = 12.5% (17 patients)
AVERAGE WEIGHT LOSS ≥ 22 WEEKS ON Rx = 15.3% (8 patients)
AVERAGE BASELINE BMI = 33.6

A novel combination therapy is described for the treatment of obese or overweight patients that can result in weight losses of greater than 5-10%, perhaps even as great as 15-20%. The therapy combines phentermine or a phentermine-like drug with drug previously recognized for the treatment of epileptic seizures, known as topiramate. The combination therapy results in greater initial weight loss than other recognized therapies, potential greater overall weight loss, and can be continued for significant periods of time with fewer and less serious side effects than other recognized weight loss treatments. In particular, the combination therapy far surpasses the modest anorexient effects of phentermine monotherapy and can be continued for significant periods of time without the loss of effectiveness experienced by patients being treated with phentermine alone. Moreover, the combination therapy has been found to ameliorate symptoms associated with Syndrome X and accordingly, has potential use in the treatment of Syndrome X.

## Claims

1. Use of a combination of a sympathomimetic agent and an anticonvulsant sulfamate derivative for the preparation of a medicament for effecting weight loss in a subject, the sympathomimetic agent being phenylethylamine, epinephrine, norepinephrine, epinine, dopamine, dabutamine, nordefrin, ethylnorepinephrine, isoproterenol, protokylol, isoetharine, metaproterenol, terbutaline, metaraminol, phenylephrine, tyramine, hydroxyamphetamine, methoxyphenamine, methoxamine, albuterol, amphetamine, methamphetamine, benzphetamine, ephedrine, phenylpropanolamine, mephentermine, phentermine, chlorphentermine, fenfluramine, tuaminoheptane, propylhexedrine, diethylpropran, phenmetrazine, or phendimetrazine, and the anticonvulsant sulfamate derivative is a compound of the formula I: 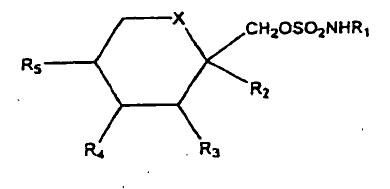 wherein
X is CH₂ or oxygen;
R₁ is hydrogen or alkyl; and
R₂, R₃, R₄ and R₅ are independently hydrogen or C₁-C₄ alkyl; when X is oxygen, R₂ and R₃ and/or R₄ and R₅ together may be a methylenedioxy group of the following formula II: 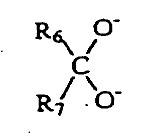 wherein
R₆ and R₇ are the same or different and are hydrogen, C₁-C₃ alkyl or may be taken together to form a cyclopentyl or cyclohexyl ring.

2. Use of claim 1, wherein the sympathomimetic agent is amphetamine, methamphetamine, benzphetamine, phyenylpropanolamine, phentermine, chlorphentermine, diethylpropran, phenmetrazine or phendimetrazine.

3. Use of claim 2, wherein the sympathomimetic agent is phentermine.

4. Use of claim 1 wherein the anticonvulsant sulfamate derivative is topiramate.

5. Use according to claim 1 of a combination of phentermine and topiramate for the preparation of a medicament for effecting weight loss in a subject.

6. Use of claim 5, wherein the therapeutically effective amount of phentermine is from 5 to 60 mg daily.

7. Use of claim 5, wherein the amount of topiramate is from 50 to 1500 mg daily.

8. Use of claim 7, wherein the amount of topiramate is less than 400 mg daily.

9. Use of claim 8, wherein the amount of topiramate is from 50 to 400 mg daily.

10. Use of claim 5, wherein the amount of phentermine is 15 mg daily and the amount of topiramate is less than 400 mg daily.

11. The use of any one of claims 1 to 10, wherein the subject is overweight.

12. The use of any one of claims 1 to 10, wherein the subject is obese.

13. The use of any one of claims 1 to 10, wherein the subject is neither overweight nor obese.

14. The use of claim 3, wherein the subject suffers from a condition that can be alleviated with loss of body weight.

15. The use of claim 5, wherein the phentermine and the topiramate are adapted for simultaneous administration.

16. The use of claim 15, wherein the phentermine and the topiramate are contained in a single pharmaceutical formulation.

17. The use of claim 5, wherein the phentermine and the topiramate are adapted for separate administration.

18. The use of claim 17, wherein the phentermine and the topiramate are adapted for administration at different times of the day.

19. The use of claim 5, wherein the phentermine is for administration in the morning and the topiramate is for administration at least once later in the day.

20. The use of claim 5, wherein the amount of topiramate administered to the subject is gradually increased, over an extended time period, from an initial daily dosage up to a final daily dosage suitable for continued therapy.

21. The use of claim 5, wherein the phentermine is contained in an immediate release dosage form.

22. The use of claim 21, wherein the topiramate is contained in an immediate release dosage form or a controlled release dosage form.

23. The use of claim 5, wherein the phentermine and the topiramate are adapted for oral administration.

24. Use of a sympathomimetic agent for the preparation of a medicament for effecting weight loss in a subject in combination with an anticonvulsant sulfamate derivative,
the sympathomimetic agent being phenylethylamine, epinephrine, norepinephrine, epinine, dopamine, dabutamine, nordefrin, ethylnorepinephrine, isoproterenol, protokylol, isoetharine, metaproterenol, terbutaline, metaraminol, phenylephrine, tyramine, hydroxyamphetamine, methoxyphenamine, methoxamine, albuterol, amphetamine, methamphetamine, benzphetamine, ephedrine, phenylpropanolamine, mephentermine, phentermine, chlorphentermine, fenfluramine, tuaminoheptane, propylhexedrine, diethylpropran, phenmetrazine, or phendimetrazine, and the anticonvulsant sulfamate derivative being of the formula I: 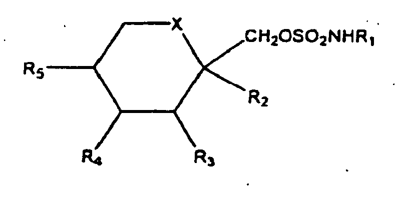 wherein
X is CH₂ or oxygen;
R₁ is hydrogen or alkyl; and
R₂, R₃, R₄ and R₅ are independently hydrogen or C₁-C₄ alkyl; when X is oxygen, R₂ and R₃ and/or R₄ and R₅ together may be a methylenedioxy group of the following formula II: 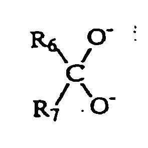 wherein
R₆ and R₇ are the same or different and are hydrogen, C₁-C₃ alkyl or may be taken together to form a cyclopentyl or cyclohexyl ring.

25. Use of an anticonvulsant sulfamate derivative for the preparation of a medicament for effecting weight loss in a subject in combination with a sympathomimetic agent, the sympathomimetic agent being phenylethylamine, epinephrine, norepinephrine, epinine, dopamine, dabutamine, nordefrin, ethylnorepinephrine, isoproterenol, protokylol, isoetharine, metaproterenol, terbutaline, metaraminol, phenylephrine, tyramine, hydroxyamphetamine, methoxyphenamine, methoxamine, albuterol, amphetamine, methamphetamine, benzphetamine, ephedrine, phenylpropanolamine, mephentermine, phentermine, chlorphentermine, fenfluramine, tuaminoheptane, propylhexedrine, diethylpropran, phenmetrazine, or phendimetrazine and the anticonvulsant sulfamate derivative being of the formula I: 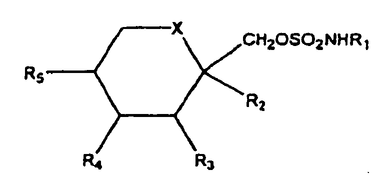 wherein
X is CH₂ or oxygen;
R₁ is hydrogen or alkyl; and
R₂, R₃, R₄ and R₅ are independently hydrogen or C₁-C₄ alkyl; when X is oxygen, R₂ and R₃ and/or R₄ and R₅ together may be a methylenedioxy group of the following formula II: 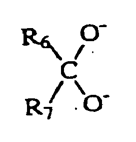 wherein
R₆ and R₇ are the same or different and are hydrogen, C₁-C₃ alkyl or may be taken together to form a cyclopentyl or cyclohexyl ring.

26. A product containing an anticonvulsant sulfamate derivative as defined in claim 1 and a sympathomimetic agent as defined in claim 1 as a combined preparation for simultaneous or sequential use for effecting weight loss in a subject.

27. A pharmaceutical composition comprising topiramate and phentermine.

## Patentansprüche

1. Verwendung einer Kombination eines Sympathomimetikums und eines krampflösenden Sulfamatderivats zur Herstellung eines Arzneimittels zur Bewirkung eines Gewichtsverlusts bei einem Subjekt, wobei das Sympathomimetikum Phenylethylamin, Epinephrin, Norepinephrin, Epinin, Dopamin, Dabutamin, Nordefrin, Ethylnorepinephrin, Isoproterenol, Protokylol, Isoetharin, Metaprotereonl, Terbutalin, Metaraminol, Phenylephrin, Tyramin, Hydroxyamphetamin, Methoxyphenamin, Methoxamin, Albuterol, Amphetamin, Methamphetamin, Benzphetamin, Ephedrin, Phenylpropanolamin, Mephentermin, Phentermin, Chlorphentermin, Fenfluramin, Tuaminoheptan, Propylhexedrin, Diethylpropran, Phenmetrazin oder Phendimetrazin ist und das krampflösende Sulfamatderivat eine Verbindung der Formel I ist: 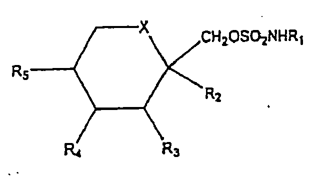 worin
X CH₂ oder Sauerstoff ist;
R₁ Wasserstoff oder Alkyl ist; und
R₂, R₃, R₄ und R₅ unabhängig Wasserstoff oder C₁-C₄-Alkyl sind; wenn X Sauerstoff ist, R₂ und R₃ und/oder R₄ und R₅ zusammen eine Methylendioxygruppe der folgenden Formel II sein können: 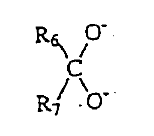 worin
R₆ und R₇ dasselbe oder verschieden sind und für Wasserstoff oder C₁-C₃-Alkyl stehen oder unter Bildung eines Cyclopentyl-oder Cyclohexylrings zusammengenommen sein können.

2. Verwendung nach Anspruch 1, wobei das Sympathomimetikum Amphetamin, Methamphetamin, Benzphetamin, Phenylpropanolamin, Phentermin, Chlorphentermin, Diethylpropran, Phenmetrazin oder Phendimetrazin ist.

3. Verwendung nach Anspruch 2, wobei das Sympathomimetikum Phentermin ist.

4. Verwendung nach Anspruch 1, wobei das krampflösende Sulfamatderivat Topiramat ist.

5. Verwendung nach Anspruch 1 einer Kombination von Phentermin und Topiramat zur Herstellung eines Arzneimittels zur Bewirkung von Gewichtsverlust bei einem Subjekt.

6. Verwendung nach Anspruch 5, wobei die therapeutisch wirksame Menge an Phentermin 5 bis 60 mg täglich beträgt.

7. Verwendung nach Anspruch 5, wobei die Menge an Topiramat 50 bis 1500 mg täglich beträgt.

8. Verwendung nach Anspruch 7, wobei die Menge an Topiramat weniger als 400 mg täglich beträgt.

9. Verwendung nach Anspruch 8, wobei die Menge an Topiramat 50 bis 400 mg täglich beträgt.

10. Verwendung nach Anspruch 5, wobei die Menge an Phentermin 15 mg täglich beträgt und die Menge an Topiramat weniger als 400 mg täglich beträgt.

11. Verwendung nach einem der Ansprüche 1 bis 10, wobei das Subjekt übergewichtig ist.

12. Verwendung nach einem der Ansprüche 1 bis 10, wobei das Subjekt fettleibig ist.

13. Verwendung nach einem der Ansprüche 1 bis 10, wobei das Subjekt weder übergewichtig noch fettleibig ist.

14. Verwendung nach Anspruch 3, wobei das Subjekt unter einem Zustand leidet, der mit einem Verlust an Körpergewicht gemindert werden kann.

15. Verwendung nach Anspruch 5, wobei das Phentermin und das Topiramat zur gleichzeitigen Verabreichung angepasst sind.

16. Verwendung nach Anspruch 15, wobei das Phentermin und das Topiramat in einer einzelnen pharmazeutischen Formulierung enthalten sind.

17. Verwendung nach Anspruch 5, wobei das Phentermin und das Topiramat zur getrennten Verabreichung angepasst sind.

18. Verwendung nach Anspruch 17, wobei das Phentermin und das Topiramat zur Verabreichung zu verschiedenen Tageszeiten angepasst sind.

19. Verwendung nach Anspruch 5, wobei das Phentermin zur Verabreichung am Morgen ist und das Topiramat zur Verabreichung zumindest einmal später am Tag ist.

20. Verwendung nach Anspruch 5, wobei die Menge an dem Subjekt verabreichten Topiramat schrittweise über einen ausgedehnten Zeitraum von einer anfänglichen täglichen Dosierung bis zu einer endgültigen täglichen Dosierung, die zur kontinuierlichen Therapie geeignet ist, erhöht wird.

21. Verwendung nach Anspruch 5, wobei das Phentermin in einer Dosierungsform mit sofortiger Freisetzung enthalten ist.

22. Verwendung nach Anspruch 21, wobei das Topiramat in einer Dosierungsform mit sofortiger Freisetzung oder einer Dosierungsform mit kontrollierter Freisetzung enthalten ist.

23. Verwendung nach Anspruch 5, wobei das Phentermin und das. Topiramat zur oralen Verabreichung angepasst sind.

24. Verwendung eines Sympathomimetikums zur Herstellung eines Arzneimittels zur Bewirkung von Gewichtsverlust bei einem Subjekt in Kombination mit einem krampflösenden Sulfamat derivat,
wobei das Sympathomimetikum Phenylethylamin, Epinephrin, Norepinephrin, Epinin, Dopamin, Dabutamin, Nordefrin, Ethylnorepinephrin, Isoproterenol, Protokylol, Isoetharin, Metaproterenol, Terbutalin, Metaraminol, Phenylephrin, Tyramin, Hydroxyamphetamin, Methoxyphenamin, Methoxamin, Albuterol, Amphetamin, Methamphetamin, Benzphetamin, Ephedrin, Phenylpropanolamin, Mephentermin, Phentermin, Chlorphentermin, Fenfluramin, Tuaminoheptan, Propylhexedrin, Diethylpropran oder Phenmetrazin ist und das krampflösende Sulfamatderivat die Formel I besitzt: 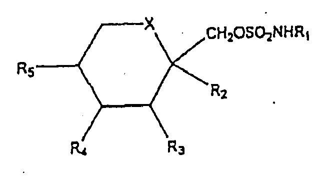 worin
X CH₂ oder Sauerstoff ist;
R₁ Wasserstoff oder Alkyl ist; und
R₂, R₃, R₄ und R₅ unabhängig Wasserstoff oder C₁-C₄-Alkyl sind; wenn X Sauerstoff ist, R₂ und R₃ und/oder R₄ und R₅ zusammen eine Methylendioxygruppe der folgenden Formel II sein können: 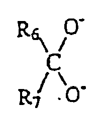 worin
R₆ und R₇ dasselbe oder verschieden sind und Wasserstoff oder C₁-C₃-Alkyl sind oder unter Bildung eines Cyclopentyl- oder Cyclohexylrings zusammengenommen sein können.

25. Verwendung eines krampflösenden Sulfamatderivats zur Herstellung eines Arzneimittels zur Bewirkung von Gewichtsverlust bei einem Subjekt in Kombination mit einem Sympathomimetikum, wobei das Sympathomimetikum Phenylethylamin, Epinephrin, Norepinephrin, Epinin, Dopamin, Dabutamin, Nordefrin, Ethylnorepinephrin, Isoproterenol, Protokylol, Isoetharenol, Metaproterenol, Terbutalin, Metaraminol, Phenylephrin, Tyramin, Hydroxyamphetamin, Methoxyphenamin, Methoxamin, Albuterol, Amphetamin, Methamphetamin, Benzphetamin, Ephedrin, Phenylpropanolamin, Mephentermin, Phentermin, Chlorphentermin, Fenfluramin, Tuaminoheptan, Propylhexedrin, Diethylpropan oder Phenmetrazin ist und das krampflösende Sulfamatderivat die Formel I besitzt: 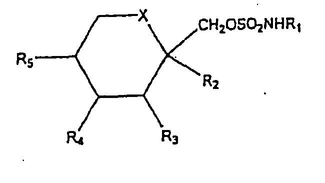 worin
X CH₂ oder Sauerstoff ist;
R₁ Wasserstoff oder Alkyl ist; und
R₂, R₃, R₄ und R₅ unabhängig Wasserstoff oder C₁-C₄-Alkyl sind; wenn X Sauerstoff ist, R₂ und R₃ und/oder R₄ und R₅ zusammen eine Methylendioxygruppe der folgenden Formel II sein können: 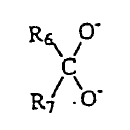 worin
R₆ und R₇ dasselbe oder verschieden sind und Wasserstoff oder C₁-C₃-Alkyl sind oder unter Bildung eines Cyclopentyl- oder Cyclohexylrings zusammengenommen sein können.

26. Produkt, das ein krampflösendes Sulfamatderivat wie in Anspruch 1 definiert und ein Sympathomimetikum wie in Anspruch 1 definiert als ein Kombinationspräparat zur gleichzeitigen oder aufeinander folgenden Verwendung zur Bewirkung von Gewichtsverlust bei einem Subjekt enthält.

27. Pharmazeutische Zusammensetzung, die Topiramat und Phentermin umfasst.

## Revendications

1. Utilisation d'une combinaison d'un agent sympathomimétique et d'un dérivé de sulfamate anticonvulsivant pour la préparation d'un médicament agissant sur la perte de poids chez un sujet, l'agent sympathomimétique étant une phényléthylamine, une épinéphrine, une norépinéphrine, une épinine, une dopamine, une dabutamine, une nordéfrine, une éthylnorépinéphrine, un isoprotérénol, un protokylol, une isoétharine, un métaprotérénol, une terbutaline, un métaraminol, une phényléphrine, une tyramine, une hydroxyamphétamine, une méthoxyphénamine, une méthoxamine, un albutérol, une amphétamine, une méthamphétamine, une benzophétamine, une éphédrine, une phénylpropanolamine, une mephentamine, une phentermine, une chlorophentermine, une fenfluramine, un tuaminoheptane, une propylhexédrine, une diéthylpropran, une phenmétrazine ou une phendimétrazine, et le dérivé de sulfamate anticonvulsivant est un composé de formule I : 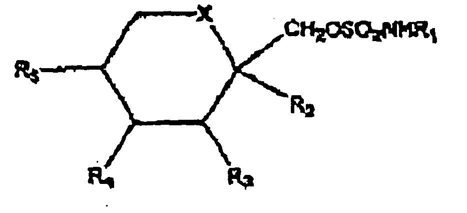 dans laquelle
X représente CH₂ ou un atome d'oxygène ;
R₁ représente un atome d'hydrogène ou un groupe alkyle ; et
R₂, R₃, R₄ et R₅ sont indépendamment un atome d'hydrogène ou un groupe alkyle en C₁-C₄ ; quand X représente un atome d'oxygène, R₂ et R₃ et/ou R₄ et R₅ ensemble peuvent représenter un groupe méthylènedioxy répondant à la formule II suivante : 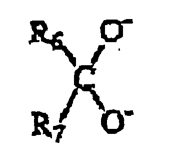 dans laquelle
R₆ et R₇ sont identiques ou différents et représentent un atome d'hydrogène, un groupe alkyle en C₁-C₃ ou peuvent être pris ensemble pour former un noyau cyclohexyle ou cyclopentyle.

2. Utilisation selon la revendication 1, dans laquelle l'agent sympathomimétique est une amphétamine, une méthamphétamine, une benzophétamine, une phénylpropanolamine, une phentermine, une chlorophentermine, un diéthylpropran, une phenmétrazine ou une phendimétrazine.

3. Utilisation selon la revendication 2, dans laquelle l'agent sympathomimétique est la phentermine.

4. Utilisation selon la revendication 1, dans laquelle le dérivé de sulfamate anticonvulsivant est le topiramate.

5. Utilisation selon la revendication 1 d'une combinaison de phentermine et de topiramate pour la préparation d'un médicament agissant sur la perte de poids chez un sujet.

6. Utilisation selon la revendication 5, dans laquelle la quantité thérapeutiquement efficace de phentermine s'inscrit entre 5 et 60 mg par jour.

7. Utilisation selon la revendication 5, dans laquelle la quantité de topiramate s'inscrit entre 50 et 1 500 mg par jour.

8. Utilisation selon la revendication 7, dans laquelle la quantité de topiramate est inférieure à 400 mg par jour.

9. Utilisation selon la revendication 8, dans laquelle la quantité de topiramate s'inscrit entre 50 et 400 mg par jour.

10. Utilisation selon la revendication 5, dans laquelle la quantité de phentermine s'élève à 15 mg par jour et la quantité de topiramate est inférieure à 400 mg par jour.

11. Utilisation selon l'une quelconque des revendications 1 à 10, dans laquelle le sujet présente une surcharge pondérale.

12. Utilisation selon l'une quelconque des revendications 1 à 10, dans laquelle le sujet est obèse.

13. Utilisation selon l'une quelconque des revendications 1 à 10, dans laquelle le sujet ne présente pas de surcharge pondérale et n'est pas obèse.

14. Utilisation selon la revendication 3, dans laquelle le sujet souffre d'une pathologie qui peut être soulagée par la perte de poids corporel.

15. Utilisation selon la revendication 5, dans laquelle la phentermine et le topiramate sont adaptés pour une administration concomitante.

16. Utilisation selon la revendication 15, dans laquelle la phentermine et le topiramate sont contenus dans une formulation pharmaceutique unique.

17. Utilisation selon la revendication 5, dans laquelle la phentermine et le topiramate sont adaptés pour une administration séparée.

18. Utilisation selon la revendication 17, dans laquelle la phentermine et le topiramate sont adaptés pour une administration à des moments différents de la journée.

19. Utilisation selon la revendication 5, dans laquelle la phentermine est appropriée pour une administration le matin et le topiramate pour une administration au moins une fois dans la journée et ce, ultérieurement.

20. Utilisation selon la revendication 5, dans laquelle la quantité de topiramate administrée chez un sujet est augmentée progressivement, sur une période de temps étendue, allant d'un dosage quotidien initial à un dosage quotidien final approprié à une utilisation dans une thérapie suivie.

21. Utilisation selon la revendication 5, dans laquelle la phentermine est contenue dans une forme galénique à libération immédiate.

22. Utilisation selon la revendication 21, dans laquelle le topiramate est contenu dans une forme galénique à libération immédiate ou dans une forme galénique à libération contrôlée.

23. Utilisation selon la revendication 5, dans laquelle la phentermine et le topiramate sont adaptés pour une administration par voie orale.

24. Utilisation d'un agent sympathomimétrique pour la préparation d'un médicament agissant sur la perte de poids chez un sujet en combinaison avec un dérivé de sulfamate anticonvulsivant, l'agent sympathomimétique étant une phényléthylamine, une épinéphrine, une norépinéphrine, une épinine, une dopamine, une dabutamine, une nordéfrine, une éthylnorépinéphrine, un isoprotérénol, un protokylol, une isoétharine, un métaprotérénol, une terbutaline, un métaraminol, une phényléphrine, une tyramine, une hydroxyamphétamine, une méthoxyphénamine, une méthoxamine, un albutérol, une amphétamine, une méthamphétamine, une benzophétamine, une éphédrine, une phénylpropanolamine, une méphentermine, une phentermine, une chlorophentermine, une fenfluramine, un tuaminoheptane, une propylhexédrine, une diéthylpropran, une phenmétrazine ou une phendimétrazine et le dérivé de sulfamate anticonvulsivant répondant à la formule I : 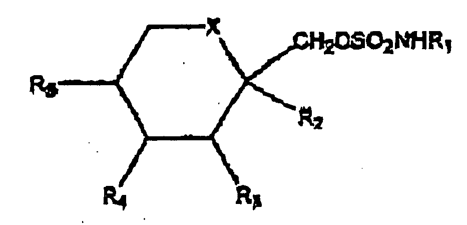 dans laquelle
X représente CH₂ ou un atome d'oxygène ;
R₁ représente un atome d'hydrogène ou un groupe alkyle ; et
R₂, R₃, R₄ et R₅ sont indépendamment un atome d'hydrogène ou un groupe alkyle en C₁-C₄ ; quand X représente un atome d'oxygène, R₂ et R₃ et/ou R₄ et R₅ ensemble peuvent représenter un groupe méthylènedioxy répondant à la formule II suivante : 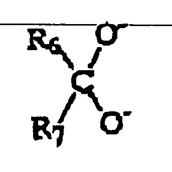 dans laquelle
R₆ et R₇ sont identiques ou différents et représentent un atome d'hydrogène, un groupe alkyle en C₁-C₃ ou peuvent être pris ensemble pour former un noyau cyclohexyle ou cyclopentyle.

25. Utilisation d'un dérivé de sulfamate anticonvulsivant pour la préparation d'un médicament agissant sur la perte de poids chez un sujet en combinaison avec un agent sympathomimétique, l'agent sympathomimétique étant une phényléthylamine, une épinéphrine, une norépinéphrine, une épinine, une dopamine, une dabutamine, une nordéfrine, une éthylnorépinéphrine, un isoprotérénol, un protokylol, une isoétharine, un métaprotérénol, une terbutaline, un métaraminol, une phényléphrine, une tyramine, une hydroxyamphétamine, une méthoxyphénamine, une méthoxamine, un albutérol, une amphétamine, une méthamphétamine, une benzophétamine, une éphédrine, une phénylpropanolamine, une méphentermine, une phentermine, une chlorophentermine, une fenfluramine, un tuaminoheptane, une propylhexédrine, une diéthylpropran, une phenmétrazine ou une phendimétrazine et le dérivé de sulfamate anticonvulsivant répondant à la formule I : 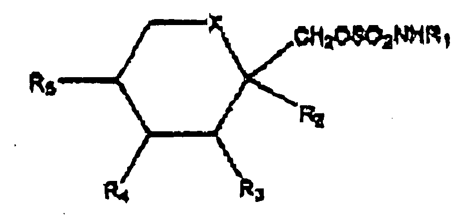 dans laquelle
X représente CH₂ ou un atome d'oxygène ;
R₁ représente un atome d'hydrogène ou un groupe alkyle ; et
R₂, R₃, R₄ et R₅ sont indépendamment un atome d'hydrogène ou un groupe alkyle en C₁-C₄ ; quand X représente un atome d'oxygène, R₂ et R₃ et/ou R₄ et R₅ ensemble peuvent être un groupe méthylènedioxy répondant à la formule II suivante : 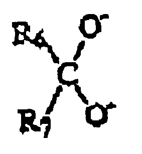 dans laquelle
R₆ et R₇ sont identiques ou différents et représentent un atome d'hydrogène, un groupe alkyle en C₁-C₃ ou peuvent être pris ensemble pour former un noyau cyclohexyle ou cyclopentyle.

26. Produit contenant un dérivé de sulfamate anticonvulsivant selon la revendication 1 et un agent sympathomimétique selon la revendication 1 sous la forme d'une préparation combinée pour une utilisation séquentielle ou concomitante agissant sur la perte de poids chez un sujet.

27. Composition pharmaceutique comprenant le topiramate et la phentermine.
